## Europäisches Patentamt
## European Patent Office
## Office européen des brevets

(11) Publication number: **0 423 155 B1**

## EUROPEAN PATENT SPECIFICATION

(45) Date of publication of patent specification: **02.11.94**  (51) Int. Cl.5: **A61L  27/00**

(21) Application number: **89906783.9**

(22) Date of filing: **12.06.89**

(86) International application number:
**PCT/FI89/00105**

(87) International publication number:
**WO 90/00410 (25.01.90 90/03)**

(54) **REINFORCED POLYMERIC BIOMATERIAL FILMS.**

(30) Priority: **05.07.88 FI 883197**

(43) Date of publication of application:
**24.04.91 Bulletin  91/17**

(45) Publication of the grant of the patent:
**02.11.94 Bulletin  94/44**

(84) Designated Contracting States:
**AT BE CH DE FR GB IT LI LU NL SE**

(56) References cited:
**EP-A- 0 146 398**
**EP-A- 0 204 931**
**US-A- 4 731 074**

**Dialog Information Services, File 155, Medline 82105985, Dialog accession no. 04562985; J. PILET et al.: "Comparison of poly(dG-dC).poly(dG-dC9 conformations in oriented films and in solution", Proc Natl Acad Sci US, Jan. 1982, 79(1), pp. 26-30**

(73) Proprietor: **BIOCON OY**
**Runeberginkatu 3 A 1**
**SF-33710 Tampere (FI)**

(72) Inventor: **TÖRMÄLÄ, Pertti**
**Runeberginkatu 3 A**
**SF-33710 Tampere (FI)**
Inventor: **VAINIONPÄÄ, Seppo**
**Orapihlajatie 21-27 B 12**
**SF-00320 Helsinki (FI)**

(74) Representative: **Smulders, Theodorus A.H.J., Ir. et al**
**Vereenigde Octrooibureaux**
**Nieuwe Parklaan 97**
**NL-2587 BN 's-Gravenhage (NL)**

Rank Xerox (UK) Business Services
(3. 10/3.0 9/3.3.3)

Rapra Abstracts, abstract no. 7721808L; U.R. SHETTIGAR: "Collagen film for burn wounds dressings reconstituted from animal intestines", Artificial Organs 6, no. 3, Aug 1982, pp. 256-60

Chemical Abstracts, vol. 108 (1988), abstract no. 156432b; T. NAKAMURA: Adv. Biomater., 7(Biomater.Clin. Appl.), pp. 759-64, 1987

## Description

The invention relates to an absorbable and/or soluble polymeric film.

Polymeric biomaterials like films (or membranes) have several application possibilities in surgical treatment. Porous or nonporous polymer film can be applied e.g. to support from outside an operated or damaged tissue or organ or their part e.g. by fixing the supporting film by tissue adhesive or by suturing around the operated internal organ. Porous or nonporous film can be applied also to separate inflamed tissues or organs or their parts from their environment and in this way to prevent the spreading of infection. Separating films can be applied also to separate cell tissues from each others and at the same time to guide in a controlled way the growth of the cells. Such a typical application is the use of polymer film to separate infected, surgically cleaned root of the tooth from the gingival connective tissue and epithelium. Then the periodontal ligamentum -and cementum tissue can grow into the coronal direction on the surface of the healing root, which leads to a new fixation of the tooth. In this way the periodontal structures can be regenerated (J. Gottlow, S. Nyman, J. Lindhe, T. Karring and J. Wennström, J. Clin. Periodontol., 13 (1986) 204). After a periodontal surgical operation there are four types cell tissues which try to cover the surface of the root of the tooth. In an uncontrolled situation the epithelium grows first along the surface of the root and prevents the refixation of the tooth. Also the connective tissue of the gingiva can fix on the surface of the root. However, without the cementum or periodontal ligament the fixation is weak and can lead to the breaking of the fixation (S. Nyman, T. Karring, J. Lindhe and S. Planten, J. Clin. Periodontol., 7 (1980) 394; T. Karring, F. Isidor, S. Nyman and J. Lindhe, J. Clin. Periodontol., 12 (1985) 51).

The cells which produce cementum and periodontal ligament grow so slowly that they usually don't grow on the surface of the infected root of the tooth before gingival tissue and epithelium. However, the studies of guided tissue regeneration have shown that cells, which produce cementum, can grow on the surface of the root, if the surface of the root has been isolated of other tissues during the healing (S. Nyman, J. Gottlow, T. Karring and J. Lindhe, J. Clin. Periodontol., 9 (1982) 275).

In surgical use are known biostable films, which act as a protecting layer between the gingival connective tissue and the root of the tooth. Such films form a protected space on the defect of the root. In this space the remaining cells of periodontal ligament can cover selectively the surface of the root. E.g. Gore-Tex is such a biostable film material. It is polytetrafluoroethylene (PTFE) film, which comprises PTFE nodules and fine fibrils which connect the nodules to each others. Such biostable films or membranes must, however, be removed with an other operation after the healing of the root of the tooth. The removal operation must be carried out typically 1-3 months after the first operation. This means significant economical costs and additional risks (e.g. infections) to the patient.

An ideal film or membrane to protect the growth of the cells which produce cementum and periodontal ligament on the root of the tooth, is an absorbable and/or soluble (biodegradable) film or membrane, which is digested by the metabolism of the living cells and/or by the solution without causing tissue reactions which should prevent the healing. In this case the need of the second operation is eliminated.

Among biomaterials experts it is known to use melt-molded or solution evaporated absorbable films to separate tissues, organs or their parts from each others or to support operated or damaged tissues or organs or their parts from their outer surface.

Accordingly it is known to use polylactide film which has been manufactured of chloroform solutions of polylactide by evaporation, to separate the healing surface of the root of the tooth from the gingival connective tissue and the epithelium. In such a situation the periodontal ligamentum- and cementum tissue can grow into the coronal direction under the protection of the polylactide film, which leads to a new connective tissue fixation (I. Magnusson, C. Batich and B.R. Collins, J. Periodontol., January, 1988, p. 1). Such polymer films are, however, weak of their mechanical strength (tensile strength typically 40-60 MPa). This leads to practical difficulties in the surgical use of such films. Such difficulties are e.g.

- Mechanically weak films break or tear easily when they are installed into the living tissue in a surgical operation. On the other hand, if the surgeon wants to secure that such a film doesn't break or tear during the operation, thick films (the thickness typically 50-500 m) must be used. Such films are stiff and therefore it is difficult to put the film tightly on the surface of the root. On the other hand, such films form a considerable amount of foreign material in the tissues, which may lead to a foreign body reaction, which may delay, disturb or prevent healing at least partially.
- The fixation of mechanically weak film on the surface of the root of the tooth by e.g. sutures which penetrate the film is difficult, because the suture can cut the weak film easily when it is drawn through the hole in the film. This may lead to tearing of the film and to the loosening of the suture during the operation.

The main reason to the poor mechanical strength properties of absorbable polymeric films which are manufactured by melt-molding or solution evaporation is that when the melt solidifies or the solution evaporates the crystallizating polymer transfomrs to a partially crystalline, spherulitic structure. Accordingly the partially crystalline, absorbable film, which has been manufactured by melt-molding or by evaporating, comprises typically folded molecular chain lamellae (the thickness 100-300 Å, the breadth about 1 $\mu$m), which are surrounded by amorphous polymer. The lamellae, on the other hand, are formed of mosaic-like folded chain blocks (the breadth a few hundred Å). The lamellae join typically to ribbon-like structures, which grow from the crystallization nuclei into three-dimensional sphere-like spherulitic structures. Because polymeric materials with spherulitic structure usually do not show significant orientation of polymeric chains in some special direction, the strength properties of such polymer materials are typically quite modest (e.g. tensile strength typically 20-60 MPa).

In this invention it has been found unexpectedly, as stated in the characteristic part of the independent claim, that when an absorbable and/or soluble film (or membrane) is reinforced with at least partially absorbable and/or soluble oriented structural elements, said orientation of the structural reinforcement elements being selected from biaxial orientation, woven fabric, knit fabric and non-woven felt, one can create new strong and tough absorbable and/or soluble films, which have at least partially oriented structure, and which can be used better than the known materials to support or to join tissues or organs or their parts and/or to separate them from each others. Such films can be used e.g. as a separating film on the surface of the root of the tooth to protect the controlled growth of periodontal ligamentum- and/or cementum tissue.

Biodegradable materials reinforced with reinforcement elements are known from EP 146398. The reinforcement elements are ceramic fibers which are oriented in a parallel way. Fibers made of ceramic materials are rather brittle, thereby the prostheses made with these cannot be bent without losing the operational characteristics of the material. Due to the stiff and brittle nature of the ceramic fibers which do not overlap, the films produced by EP 146398 are extremely anisotropic in their mechanical strength: in the direction perpendicular to the long axis of the fibers the tensile strength of the films is only 10-20% of the tensile strength in the direction of the fibers. This makes these films unsuitable for many practical applications in surgery.

EP 204931 describes surgical osteosynthesis composite material. The demands of good osteosynthesis are anatomic reduction and stability but at same time active movement of the operated limb soon after operation. Therefore, it is self evident for surgeons that osteosynthesis devices like plates, balks, rods, nails, pins, screws, etc., must be very strong and stiff and therefore films or membranes are not osteosynthesis materials or devices.

Further this invention describes a method, as stated in the characteristic part of the independent claim related to the method, to manufacture at least partially oriented, absorbable films by orientation at least part of the structural reinforcement elements of the material into the desired direction by means of the flow of the material and/or by mechanical deformation or by reinforcing the continuous or noncontinuous matrix by means of absorbable and/or soluble fibers, film-fibers or by means of structures which are constructed of them.

Further in this invention has been described the use the films of the invention as surgical implants to separate tissues and/or organs and/or their parts from each others or to support them. The use can be related to protect the controlled growth of the periodontal ligament and/or cementum tissues.

The invention will now be described in detail in the following describtion. References are made to the enclosed drawings, in which

Fig. 1a  shows the transformation of a group of lamellae to a fibrillar structure,

Fig. 1b  shows the molecular structure inside the microfibrils and between the same,

Fig. 1c  shows schematically the structure of a fibrillated polymer,

Fig. 2  shows schematically structural units found in a fibrillated structure,

Fig. 3  shows a sectional view of one embodiment of the film in accordance with the invention and

Fig. 4  shows schematically the testing method used in connection with the examples.

The orientation and fibrillation of spherulitic polymer systems is a process, which has been studied extensively in connection with the manufacturing of thermoplastic fibers. E.g. The invention U.S. Pat. 3 161 709 describes a three phases drawing process, where the melt molded polypropylene filament is transformed to a fiber with high mechanical strength.

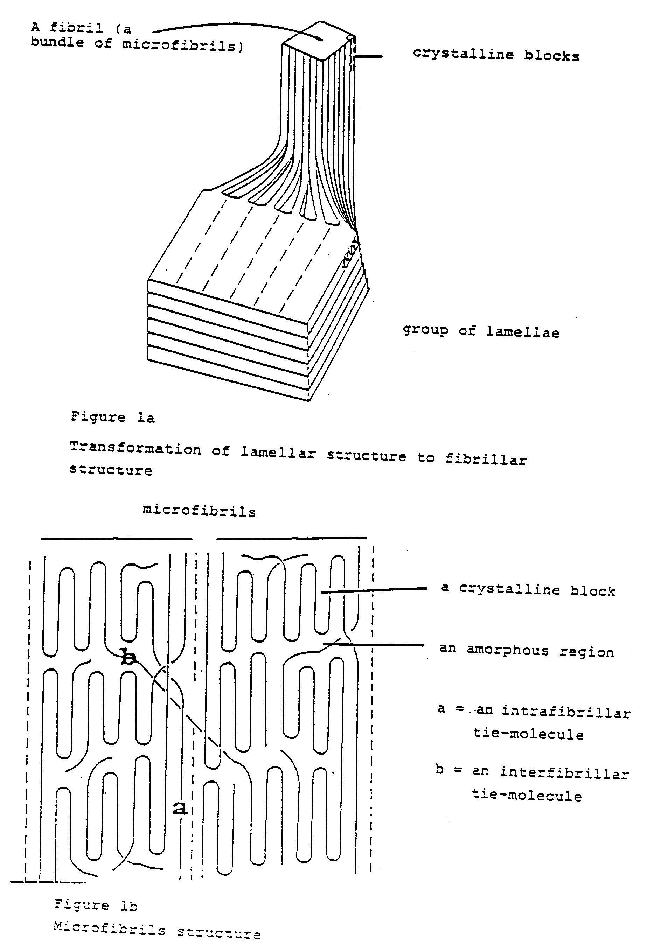

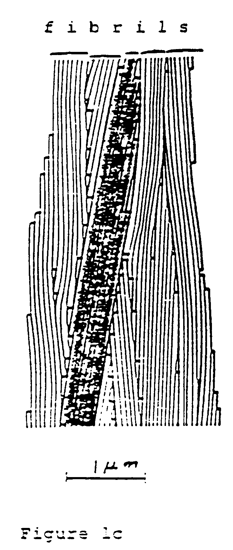

The mechanism of the fibrillation is of its main features the following one (C.L. Choy et al. Polym. Eng. Sci., 23 1983, p. 910). When a semicrystalline polymer is drawn, the molecular chains in the crystalline lamellae are aligned rapidly along the draw direction. At the same time, the spherulites are elongated and finally broken up. Crystalline blocks are torn off from the lamellae and are connected by taut tie-molecules originating from partial unfolding of chains. The alternating amorphous and crystalline regions, together with the taut tie-molecules, therefore form long thin (ca. 100 Å width) microfibrils which are aligned in the draw direction. Since the intrafibrillar tie-molecules are created at the interfaces between crystalline blocks, they lie mainly on the outside boundary of microfibrils. Tie-molecules which linked different lamellae in the starting isotropic material are now connecting different microfibrils, i.e., they become interfibrillar tie-molecules locating at the boundary layers between adjacent microfibrils. Figure 1a shows schematically, how a group of lamellae is transformed to a fibrillar structure (to a fibril which comprises a group of microfibrils) as a consequence of drawing and Figure 1b shows schematically the molecular structure inside of microfibrils and between them. Figure 1c shows schematically the structure of fibrillated polymer. This Figure shows several fibrils (one of them has been coloured grey because of clarity) which comprise several microfibrils with the length of several micrometres.

The fibrillar structure is already at relatively low draw rations λ (where λ = the length of the sample after drawing/the length of the sample before drawing). E.g. HD-polyethylene is clearly fibrillated with the λ value of 8 and polyacetal (POM) with the λ value of 3.

When the drawing of the fibrillated structure is further continued (this stage of the process is called often ultra-orientation), the fibrillar structure is deformed by shear displacement of microfibrils, giving rise to an increase in the volume fraction of extended interfibrillar tie-molecules. If the drawing is performed at high temperature, the perfectly aligned tie-molecules will be crystallized to form axial crystalline bridges connecting the crystalline blocks.

The excellent strength and elastic modulus values of the fibrillated structure are based on the strong orientation of polymer molecules and molecular segments into the direction of the drawing (into the direction of the long axis of microfibrils).

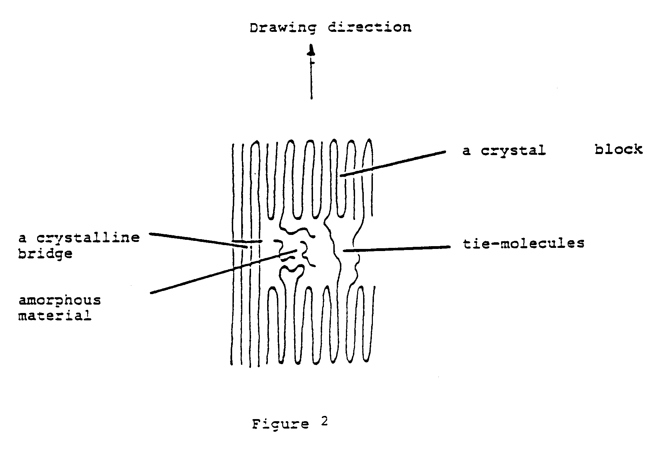

Figure 2 shows schematically following structural units which can be seen in the fibrillated structure of polymer fibers and also in the structure of macroscopical, fibrillated polymer samples like rods and tubes: crystalline blocks which are separated from each other by amorphous material (e.g. free polymer chains, chain ends and molecular folds), tie-molecules, which connect crystalline blocks with each other (the amount and thickness of tie-molecules increases with increasing draw ratio λ ) and possible crystalline bridges between crystalline blocks. Bridges can be formed during drawing when tie-molecules are oriented and grouped themselves to bridges (C.L. Choy et al. J. Polym. Sci., Polym. Phys. Ed., 19, 1981, p. 335-352). Because the oriented structure contains in the orientation direction a big amount of strong covalent bonds between the atoms of the polymer chains, such material has in the orientation direction significantly higher strength values than the nonoriented material has.

The oriented fibrillated structure which is shown in Figures 1 and 2 develops already at so-calles natural draw ratios λ < 8. When the drawing is continued after this as an ultraorientation at a high temperature, the amount of crystalline bridges can increase very high and in the extreme case bridges and crystalline blocks form a continuous crystalline structure. The effects of tie-molecules and bridges are often similar and therefore their exact discrimination from each other is not always possible.

Orientation and fibrillation can be characterized experimentally by means of several methods. The orientation function $f_c$, which can be measured by means of x-ray diffraction measurements, characterizes the orientation of molecular chains of the crystalline phase. $f_c$ Attains as a rule already at natural drawing ratios ( λ < 6) the maximum value 1. The polymeric material with spherulitic structure shows $f_c \ll 1$.

Birefringence (Δ) which can be measured by means of polarization microscope is also a quantity, which describes molecular orientation of molecular chains. As a rule it grows strongly at natural draw ratios ( λ < 6) and thereafter during ultraorientation more slowly, which shows that the molecular chains of the crystalline phase are oriented into the drawing direction at natural draw ratios and the orientation of molecules in the amorphous phase continuous further at higher draw ratios (C.L. Choy et al. Polym. Eng. Sci., 23, 1983, p. 910-922).

For unidirectionally oriented films is typical that the strength properties of the film are perpendicular to the orientation direction significantly weaker than in the orientation direction. Therefore according to an advantageous embodiment of this invention the orientation of the film is made biaxially in the direction of the plane of the film either (a) by rolling and/or drawing the thick film thinner between rollers or (b) by drawing the film simultaneously into different directions (usually in directions which are perpendicular to each others). Two-dimensional orientation creates oriented, reinforcing structural elements in the plane of the film in different directions, which makes also the strength properties of the film in perpendicular direction better.

Also other orientation methods can be applied to manufacture the films of this invention. Polymeric melt can be e.g. crystallized in a rapidly flowing state, so that the flow orientation is frozen at least partially into the solid film to reinforce it.

In addition to the above methods, where the reinforcing structural elements are created into polymer matrix during its deformation, it is also possible to apply earlier manufactured reinforcing elements to reinforce the films of the invention. Such typical structural elements are absorbable and/or soluble fibers, filaments, fibrils, film-fibers, threads, cords, non-woven structures, webs and meshes, knits, woven structures or corresponding. In these cases the absorbable and/or soluble film can be manufactured by several different methods. E.g. the reinforcing fibers or corresponding and the film forming matrix polymer can be compressed together by means of heat and pressure, which gives a film which is reinforced with fibers or with corresponding structures. It is also possible to immerse the fibers with a solution of a polymer and to evaporate the solvent at least partially and to press the fibers by means of pressure and possibly also with heat into a fiber reinforced film. It is also possible to melt the fiber construction at least partially and to compress the material into at least partially self-reinforced film. For all the previous films it is typical that because of strong, oriented structural elements these films have significantly better toughness and strength properties than the non-oriented films have.

The orientation of amorphous absorbable and/or soluble polymers does not lead to as strong increase of strength than the orientation of partially crystalline films. However, also the mechanical deformation of amorphous films leads to the increase of their strength in the direction of the deformation, because the molecular chains are also oriented in this case. However, a specially strong reinforcing effect is obtained to amorphous absorbable films only when they are reinforced with absorbable fibers or with corresponding structures as the examples of this invention show.

Table 1 shows some absorbable and/or soluble polymers, which can be applied as raw materials (both as oriented structural elements and as film matrix) in manufacturing of the films of this invention.

The films can be nonporous or they may have pores, which can be created (a) with methods known in polymer technology, like e.g. by means of different additives like gases or easily evaporating solvents etc., (b) by constructing the film mainly of fibrous structural units or (c) by perforating the film (by making holes into the film).

EP 0 423 155 B1

## Table 1. Absorbable and/or soluble polymers

Polymer

Polyglycolide (PGA)

Copolymers of glycolide:
Glycolide/L-lactide copolymers (PGA/PLLA)
Glycolide/trimethylene carbonate copolymers (PGA/TMC)

Polylactides (PLA)

Stereocopolymers of PLA:
Poly-L-lactide (PLLA)
Poly-DL-lactide (PDLLA)
L-lactide/DL-lactide copolymers

Copolymers of PLA:
Lactide/tetramethylglycolide copolymers
Lactide/trimethylene carbonate copolymers
Lactide/δ-valerolactone copolymer
Lactide/ε-caprolactone copolymer
Polydepsipeptides
PLA/polyethylene oxide copolymers
Unsymmetrically 3,6-substituted poly-1,4-dioxane-2,5-diones

Poly-β-hydroxybutyrate (PHBA)
PHBA/δ-hydroxyvalerate copolymers (PHBA/HVA)

Poly-β-hydroxypropionate (PHPA)
Poly-p-dioxanone (PDS)
Poly-δ-valerolactone
Poly-ε-caprolactone
Methylmethacrylate-N-vinyl pyrrolidine copolymers
Polyesteramides
Polyesters of oxalic acid
Polydihydropyrans
Polyalkyl-2-cyanoacrylates
Polyurethanes (PU)
Polyvinylalcohol (PVA)
Polypeptides
Poly-β-malic acid (PMLA)
Poly-β-alkanoic acids
Polyvinylalcohol (PVA)
Polyethyleneoxide (PEO)
Chitine polymers

Reference: S. Vainionpää, P. Rokkanen and P. Törmälä, Progr. Polym. Sci., in press.

It is self-evident that also other absorbable and/or soluble polymers than those given in Table 1 can be applied in manufacturing the films of this invention.

The films of this invention can contain or in structures which form part of them can be also combined different bioactive additives like antibiotics, growth hormones, drugs, hemostatic chemicals and/or other

7

therapeutic components which have advantageous effects upon the healing of tissues.

It is possible also to combine and/or to connect to the films of this invention other materials like biostable fibers, fiber constructions, films etc. to achieve the desired effects in different surgical operations.

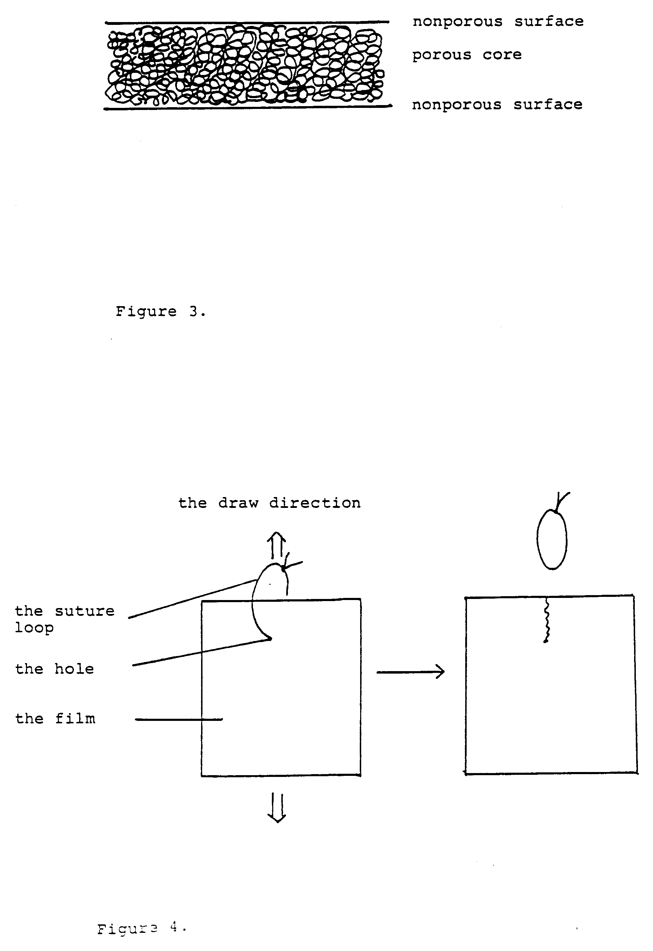

According to one advantageous embodiment the film of the invention is formed of nonporous outer surfaces and of a middle layer which contains at least partially closed porosity (Figure 3). Such a film is flexible and mechanically strong and internal porosity of the film can be at least partially filled with bioactive substances like antibiotics, drugs, growth hormones, hemostatic additives, chemotherapeutic substances etc. which can be released from the film into the surrounding tissues in a controlled way.

The invention is illustrated by means of the following nonlimiting examples.

EXAMPLE 1.

A single screw extruder (the screw diameter 25 mm) was applied to manufacture plane films of absorbable and soluble polymers and polymer mixtures by using the slot-die (the slot breadth 20 mm, the height 0.4 mm). The films were cooled with nitrogen gas blow and they were oriented biaxially either (a) by rolling them between heated rollers to the thicknesses 2 $\mu$m - 40 $\mu$m or (b) by drawing the films simultaneously in the manufacturing direction of film and in the perpendicular direction. The rolling temperatures and drawing temperatures were above the glass transition temperatures and below the melting temperatures of the materials and material mixtures. The deformation grades were between the drawing ratios 1.5 - 4. Oriented films were manufactured of absorbable and/or soluble materials given in Table 2. The tensile strengths of oriented and nonoriented corresponding films were compared to each others. From this basis was defined the relative tensile strength of the oriented films (R.T.S. = the tensile strength of the oriented film in the orientation direction/the tensile strength of the nonoriented film).

Table 2

| The relative tensile strengths (R.T.S.) and the relative tear strengths (R.Tr.S.) of oriented films. | | | |
|---|---|---|---|
| The oriented film | The thickness of the oriented film ($\mu$m) | R.T.S. | R.Tr.S. |
| PGA | 4 | 6 | 3 |
| PGA/PLLA | 20 | 5.4 | 3 |
| PGA/TMC | 40 | 3 | 2 |
| PLLA | 20 | 6 | 4 |
| PDLLA | 60 | 2 | 1.5 |
| PHBA | 50 | 3 | 2 |
| PHBA/HVA | 50 | 2 | 1.6 |
| PDS | 60 | 3 | 2 |
| PVA | 40 | 2 | 1.5 |

The tearing effect of the multifilament suture (Dexon®, size 0 (USP), manufacturer Davis & Geck, Gosport, England) upon the oriented and nonoriented films was studied by making into the film a small hole with a needle at the distance of 10 mm from the edge of the film, by drawing the suture through the hole, by knotting the suture into a loop and by tearing the suture loop through the film (see Figure 4).

The relative tear strength (R.Tr.S) of the oriented films was defined:

$$\text{R.Tr.S} = \frac{\text{Tear force of oriented film/Thickness of film}}{\text{Tear force of nonoriented film/Thickness of film}}$$

Table 2 gives the studied materials, the thicknesses of the oriented films, their relative tensile strengths and relative tear strengths.

Table 2 shows that the tensile and tear strengths of the oriented films are superior in comparison to the corresponding properties of nonoriented films.

EXAMPLE 2.

The PGA, PGA/PLLA, PGA/TMC, PLLA and PDS polymers of Table 2 were used to manufactute with so-called blow-film technique oriented absorbable films by melting the materials with a single screw extruder, by pressing the melt with pressure through a ring-like die into a tube-like preform (diameter 60 mm, the thickness of the wall 0.4 mm) and by orienting the material biaxially by means of an internal overpressure during the solidifying of the material (the thicknesses of the oriented films were between 40 $\mu$m and 80 $\mu$m).

The relative tear strengths of the oriented films had values about three.

EXAMPLE 3.

Absorbable fibers or sutures which were braided of them (thicknesses 20 $\mu$m - 400 $\mu$m) were woven into plain weave type loose fabrics. The fabric was cut into pieces (20 mm x 80 mm) and these pieces were compressed together with absorbable and/or soluble films which were manufactured of absorbable and/or soluble polymers by melting or by evaporating from solvent. During the compression the pressure (80 MPa) and heat (if necessary) was applied. The compression conditions were selected in such a way that the film material softened and/or melted and wetted the fibers. The relative tensile strengths of the fiber reinforced films (thicknesses 30 - 2000 $\mu$m) were measured in comparison to the strength values of the non-reinforced matrix polymer films (according to the Example 1). Table 3 shows the studied matrix polymers, reinforcement fibers and the relative tensile strengths (R.T.S.) of the oriented films.

The tear strengths of reinforced and non-reinforced films were studied according to the method of the Example 1. When the fiber reinforcement was made with PHBA and PHBA/HVA fibers the relative tear strengths of fiber-reinforced films were between 8 and 20. In the case of absorbable films reinforced with PGA, PGA/TMC, PGA/PLLA, PLLA and PDS fibers, the tearing suture broke before the total breaking of the film.

The measurements of Example 3 showed the superior tensile and tear strengths of the reinforced films in comparison to the non-reinforced films.

Table 3

| The structural components and the relative tensile strengths (R.T.S.) of fiber reinforced absorbable and/or soluble films. | | |
|---|---|---|
| Matrix polymer | Fiber reinforcement | R.T.S. |
| PDS | PGA | 8 |
| PDS | PGA/TMC | 6 |
| PDS | PGA/PLLA | 8 |
| PDS | PLLA | 4 |
| PDS | PHBA | 2 |
| PDS | PHBA/HVA | 1.5 |
| PDS | Chitine fiber | 6 |
| PDLLA | PGA | 12 |
| PDLLA | PGA/TMC | 8 |
| PDLLA | PGA/PLLA | 10 |
| PDLLA | PLLA | 6 |
| PDLLA | PHBA | 4 |
| PDLLA | PHBA/HVA | 3 |
| PDLLA | PDS | 1.5 |
| PLLA | PGA | 14 |
| PLLA | PGA/TMC | 6 |
| PVA | PGA | 20 |
| PVA | PGA/TMC | 14 |
| PVA | PGA/PLLA | 15 |
| PVA | PLLA | 10 |
| PVA | PHBA | 8 |
| PVA | PHBA/HVA | 6 |
| PVA | PDS | 8 |
| PVA | Chitine fibers | 6 |
| PGA/TMC | PGA | 4 |

EXAMPLE 4.

Sutures, which were braided of absorbable fibers and monofilament sutures were applied to manufacture by knitting cotton knit type knit fabric. The knit fabric was melted from its one surface by pressing it against a hot steel plate in such a way that the other surface of the knit fabric remained unmelted. The relative tensile and tear strengths of the self-reinforced absorbable films manufactured in this way were measured by comparing their tensile and tear strengths to the corresponding values of nonoriented, melt-molded corresponding films. The latter films were manufactured by melting the knit fabrics totally between two hot steel plates. The studied materials are given in Table 4. The relative tensile and tear strengths of self-reinforced films are also given in Table 4.

Table 4

| Properties of self-reinforced absorbable films | | | |
|---|---|---|---|
| Fiber material | Thickness of thread (USP) | R.T.S. of film | R.Tr.S. of film |
| PGA (Dexon) | 3-0 | 6 | * |
| PGA/TMC (Maxon) | 1 | 4 | 20 |
| PGA/PLLA (Vicryl) | 0 | 5 | * |
| PLLA | 1 | 3 | 12 |
| PDS | 1 | 3 | 8 |

*The tear thread broke before the breaking of the film.

EXAMPLE 5.

PGA-, PGA/PLLA-, PLLA- and PDLLA-fibers (thicknesses 10 - 80 $\mu$m) were cut to 20 mm long pieces. The cut fibers were collected on a porous surface by means of suction so that about 200 $\mu$m thick non-woven felts were formed. The felts were compressed mechanically (pressure 80 MPa) into tight films (or membranes) (thickness about 100 $\mu$m) and they were melted from the one surface by means of a hot plate. The relative tensile strength of the reinforced films were between 1.5 and 3 and the relative tear strengths were between 4 and 7 in comparison to the strength values of totally melted nonoriented corresponding films.

EXAMPLE 6.

PGA-, PGA/PLLA- and PLLA-fibers of Example 5 were bound together by mixing into every fiber mass 10 weight-% of finely ground PDLLA-powder (particle size about 1 $\mu$m) and by compressing fiber powder mixtures at 150 °C into porous non-woven films, where PDLLA was as a binding material forming a noncontinuous matrix. The relative tensile and tear strengths of these films was between 2 and 8 in comparison to the corresponding values of the totally melted films.

**Claims**

1. Article made of an absorbable and/or soluble polymeric reinforced composite biomaterial, characterized in that it is a film or membrane comprising absorbable and/or soluble polymeric oriented structural reinforcement elements, said orientation of the structural reinforcement elements being selected from biaxial orientation, woven fabric, knit fabric and non-woven felt.

2. A film according to claim 1, characterized in that it is at least partially porous.

3. A film according to any one of claims 1-2, characterized in that its maximum thickness is 2000 $\mu$m.

4. A film according to any one of claims 1-3, characterized in that it is manufactured at least partially of at least one of the following absorbable and/or soluble polymers: polyglycolides (PGA), polylactides (like PLLA, PDLLA), glycolide/lactide copolymers (PGA/PLA), glycolide/trimethylene carbonate copolymers (PGA/TMC), poly-$\beta$-hydroxybutyric acid (PHBA), poly-$\beta$-hydroxypropionic acid (PHPA), poly-$\delta$-hydroxyvaleric acid (PHVA), PHBA/PHVA copolymers, poly-p-dioxanone (PDS), poly-1,4-dioxanone-2,5-diones, polyesteramides (PEA), poly-$\epsilon$-caprolactone, poly-$\delta$-valerolactone, polycarbonates, polyesters of oxalic acid, glycolic esters, dihydropyrane polymers, polyetheresters, cyano-acrylates or chitine polymers.

5. A film according to any one of claims 1-4, characterized in that it has been reinforced at least partially by means of oriented structural reinforcement elements, which consist of at least one of the polymers: polyglycolides (PGA), polylactides (like PLLA, PDLLA), glycolide/lactide copolymers (PGA/PLA), glycolide/trimethylene carbonate copolymers (PGA/TMC ), poly-$\beta$-hydroxybutyric acid (PHBA ), poly-$\beta$-hydroxypropionic acid (PHPA), poly-$\delta$-hydroxyvaleric acid (PHVA), PHBA/PHVA copolymers, poly-p-dioxanone (PDS), poly-l,4-dioxanone-2,5-diones, polyesteramides (PEA), poly-$\epsilon$-caprolactone, poly-$\delta$-valerolactone, polycarbonates, polyesters of oxalic acid, glycolic esters, dihydropyrane polymers, polyetheresters, cyanoacrylates or chitine polymers.

6. A film according to any one of claims 1-5, characterized in that it includes some biofunctional chemical, like antibiotic, growth hormone, drugs etc. chemotherapeutical chemical.

7. A method to manufacture the film of any one of claims 1-6, characterized in that at least part of the structural reinforcement elements of material are oriented by means of the flow of the material and/or by means of mechanical deformation or by reinforcing the continuous or noncontinuous matrix by means of absorbable and/or soluble fibers, film fibers or by means of structures which are constructed of them.

8. Article made of an absorbable and/or soluble polymeric reinforced composite biomaterial, characterized in that it is a film or membrane for use in supporting living tissues or organs or parts of them or supporting their joining to eachother, said film or membrane comprising absorbable and/or soluble polymeric oriented structural reinforcement elements, said orientation of the structural reinforcement elements being selected from biaxial orientation, woven fabric, knit fabric and non-woven felt.

9. Article made of an absorbable and/or soluble polymeric reinforced composite biomaterial, characterized in that it is a film or membrane for use in separating from eachother living tissues or organs or parts of them, said film or membrane comprising absorbable and/or soluble polymeric oriented structural reinforcement elements, said orientation of the structural reinforcement elements being selected from biaxial orientation, woven fabric, knit fabric and non-woven felt.

10. Article made of an absorbable and/or soluble polymeric reinforced composite biomaterial, characterized in that it is a film or membrane for use in the protection of controlled growth of periodontal ligament- and/or cementum tissues, said film or membrane comprising absorbable and/or soluble polymeric oriented structural reinforcement elements, said orientation of the structural reinforcement elements being selected from biaxial orientation, woven fabric, knit fabric and non-woven felt.

**Patentansprüche**

1. Gegenstand hergestellt aus einer absorbierbaren und/oder löslichen, verstärkten polymeren Zusammensetzung aus Biomaterial, dadurch gekennzeichnet, dass es sich um einen Film oder einer Membran mit absorbierbaren und/oder löslichen strukturell orientierten polymeren Verstärkungselementen handelt, wobei es sich bei diesen Verstärkungselementen um solche aus der Gruppe mit folgenden biaxial orientierten Strukturen handelt: gewobene Ware, gewirkte Ware oder ungewobener Filz.

2. Film nach Anspruch 1, dadurch gekennzeichnet, dass dieser mindestens teilweise porig ist.

3. Film nach einer der Ansprüche 1 oder 2, dadurch gekennzeichnet, dass dessen maximale Dicke 2000 $\mu$m beträgt.

4. Film nach einem der Ansprüche 1-3, dadurch gekennzeichnet, dass dieser mindestens teilweise aus mindestens einer der folgenden absorbierbaren und/oder löslichen Polymeren gefertigt ist: Polyglycolide (PGA), Polyactide (wie PLLA, PDLLA), Glycolid/Lactide Copolymere (PGA/PLA), Glycolid/Trimtheylen Carbonat Copolymere (PGA/TMC), Poly-$\beta$-Hydroxybuttersäure (PHBA), Poly-$\beta$-Hydroxy-Propansäure (PHPA), Poly-$\delta$-Hydroxy-Pentansäure (PHVA), PHBA/PHVA Copolymere, Poly-p-Dioxanon (PDS), Poly-1.4-Dioxanon-2.5-Dione, Polyesteramid (PEA), Poly-$\epsilon$-Capronlacton, Poly-$\delta$-Valerolacton, Polycarboate, Polyester aus Oxalsäure, Glykolester, Dihydropyrane Polymere, Polyätherester, Cyan-Acrylat oder Chitin-Polymere.

5. Film nach einem der Ansprüche 1-4, dadurch gekennzeichnet, dass er mindestens teilweise mittels mindestens einem der folgenden strukturell orientierten polymeren Verstärkungselementen verstärkt ist: Polyglycolide (PGA), Polyactide (wie PLLA, PDLLA), Glycolid/Lactide Copolymere (PGA/PLA), Glycolid/Trimtheylen Carbonat Copolymere (PGA/TMC), Poly-$\beta$-Hydroxybuttersäure (PHBA), Poly-$\beta$-Hydroxy-Propansäure (PHPA), Poly-$\delta$-Hydroxy-Pentansäure (PHVA), PHBA/PHVA Copolymere, Poly-p-Dioxanon (PDS), Poly-1.4-Dioxanon-2.5-Dione, Polyesteramid (PEA), Poly-$\epsilon$-Capronlacton, Poly-$\delta$-Valerolacton, Polycarboate, Polyester aus Oxalsäure, Glykolester, Dihydropyrane Polymere, Polyätherester, Cyan-Acrylat oder Chitin-Polymere.

6. Film nach einer der Ansprüche 1-5, dadurch gekennzeichnet, dass er biofunktionelle Chemikalien, wie Antibiotikum, Wachstumshormone, Medikamente oder chemotherapeutische Chemikalien beinhaltet.

7. Ein Verfahren zur Herstellung der Films nach einem der Ansprüche 1-6, dadurch gekennzeichnet, dass die Orientierung mindestens eines Teiles der strukturierten Verstärkungselemente durch den Fluss dessen Materiales und/oder mittels mechanischer Verformung oder mittels Verstärkung der kontinuierlichen oder nicht kontinuierlichen Matrix durch absorbierbare und/oder lösliche Fasern, durch Filmfasern oder durch von diesen gebildete Strukturen erfolgt.

8. Gegenstand hergestellt aus einer absorbierbaren und/oder löslichen, verstärkten polymeren Zusammensetzung aus Biomaterial, dadurch gekennzeichnet, dass es sich um einen Film oder eine Membran handelt zur Stützung lebenden Gewebes oder Organe oder Teile davon oder zur Stützung derer

EP 0 423 155 B1

Verbindung zueinander, wobei der Film oder die Membran absorbierbare und/oder lösliche strukturell orientierte polymere Verstärkungselemente umfasst, wobei es sich bei diesen Verstärkungselementen um solche aus der Gruppe mit folgenden biaxial orientierten Strukturen handelt: gewobene Ware, gewirkte Ware oder ungewobener Filz.

9. Gegenstand hergestellt aus einer absorbierbaren und/oder löslichen, verstärkten polymeren Zusammensetzung aus Biomaterial, dadurch gekennzeichnet, dass es sich um einen Film oder eine Membran handelt zur gegenseitigen Trennung von lebenden Gewebes oder Organen oder Teilen davon, wobei der Film oder die Membran absorbierbare und/oder lösliche strukturell orientierte polymere Verstärkungselemente umfasst, wobei es sich bei diesen Verstärkungselementen um solche aus der Gruppe mit folgenden biaxial orientierten Strukturen handelt: gewobene Ware, gewirkte Ware oder ungewobener Filz.

10. Gegenstand hergestellt aus einer absorbierbaren und/oder löslichen, verstärkten polymeren Zusammensetzung aus Biomaterial, dadurch gekennzeichnet, dass es sich um einen Film oder eine Membran handelt zum Schutz des kontrollierten Wachstums von periodontalen Ligamenten und/oder Cementum-Geweben, wobei der Film oder die Membran absorbierbare und/oder lösliche strukturell orientierte polymere Verstärkungselemente umfasst, wobei es sich bei diesen Verstärkungselementen um solche aus der Gruppe mit folgenden biaxial orientierten Strukturen handelt: gewobene Ware, gewirkte Ware oder ungewobener Filz.

## Revendications

1. Article en biomatériau composite renforcé polymère absorbable et/ou soluble, caractérisé en ce qu'il s'agit d'un film ou d'une membrane comprenant des éléments de renfort structuraux orientés polymères absorbables et/ou solubles, ladite orientation des éléments de renfort structuraux étant sélectionnée parmi une orientation biaxiale, une étoffe tissée, une étoffe tricoté et un non-tissé feutre.

2. Film selon la revendication 1, caractérisé en ce qu'il est au moins partiellement poreux.

3. Film selon l'une quelconque des revendications 1 et 2, caractérisé en ce que son épaisseur maximale est de 2 000 $\mu$m.

4. Film selon l'une quelconque des revendications 1 à 3, caractérisé en ce qu'il est fabriqué au moins partiellement à partir d'au moins un des polymères absorbables et/ou solubles suivants : polyglycolides (PGA), polylactides (par exemple PLLA, PDLLA), copolymères glycolide/lactide (PGA/PLA), copolymères glycolide/carbonate de triméthylène (PGA/TMC), poly(acide $\beta$-hydroxybutyrique) (PHBA), poly(acide $\beta$-hydroxypropionique) (PHPA), poly(acide $\delta$-hydroxyvalérique) (PHVA), copolymères PHBA/PHVA, poly-p-dioxanone (PDS), poly-1,4-dioxanone-2,5-diones, polyamides d'ester (PEA), poly-$\epsilon$-caprolactone, poly-$\delta$-valérolactone, polycarbonates, polyesters de l'acide oxalique, esters glycoliques, polymères de dihydropyrane, polyétheresters, cyanoacrylates ou polymères de chitine.

5. Film selon l'une quelconque des revendications 1 à 4, caractérisé en ce qu'il est renforcé au moins partiellement au moyen d'éléments de renfort structuraux orientés, qui consistent en au moins un des polymères suivants : polyglycolides (PGA), polylactides (par exemple PLLA, PDLLA), copolymères glycolide/lactide (PGA/PLA), copolymères glycolide/carbonate de triméthylène (PGA/TMC), poly-(acide $\beta$-hydroxybutyrique) (PHBA), poly(acide $\beta$-hydroxypropionique) (PHPA), poly(acide $\delta$-hydroxyvalérique) (PHVA), copolymères PHBA/PHVA, poly-p-dioxanone (PDS), poly-1,4-dioxanone-2,5-diones, polyamides d'ester (PEA), poly-$\epsilon$-caprolactone, poly-$\delta$-valérolactone, polycarbonates , polyesters de l'acide oxalique, esters glycoliques, polymères de dihydropyrane, polyétheresters, cyanoacrylates ou polymères de chitine.

6. Film selon l'une quelconque des revendications 1 à 5, caractérisé en ce qu'il contient certains produits chimiques biofonctionnels tels qu'antibiotiques, hormones de croissance, médicaments, produits chimiothérapeutiques etc.

7. Procédé de production du film selon l'une quelconque des revendications 1 à 6, caractérisé en ce qu'au moins une partie des éléments de renfort structuraux du matériau est orientée par fluage du matériau et/ou par déformation mécanique ou par renfort de la matrice continue ou discontinue au moyen de fibres, de films-fibres absorbables et/ou solubles ou au moyen de structures constituées par ces derniers.

8. Article en biomatériau composite renforcé polymère absorbable et/ou soluble, caractérisé en ce qu'il s'agit d'un film ou d'une membrane à utiliser pour soutenir des tissus ou des organes ou des parties de tissu ou d'organe vivants ou pour favoriser leur réunion, ledit film ou membrane comprenant des éléments de renfort structuraux orientés polymères absorbables et/ou solubles, ladite orientation des éléments de renfort structuraux étant sélectionnée parmi une orientation biaxiale, une étoffe tissée, une étoffe tricotée et un non-tissé feutre.

9. Article en biomatériau composite renforcé polymère absorbable et/ou soluble, caractérisé en ce qu'il s'agit d'un film ou d'une membrane à utiliser pour séparer les uns des autres des tissus ou des organes ou des parties d'organe ou de tissu vivants, ledit film ou ladite membrane comprenant des éléments de renfort structuraux orientés polymères absorbables et/ou solubles, ladite orientation des éléments de renfort structuraux étant sélectionnée parmi une orientation biaxiale, une étoffe tissée, une étoffe tricotée et un non-tissé feutre.

10. Article en biomatériau composite renforcé polymère absorbable et/ou soluble, caractérisé en ce qu'il s'agit d'un film ou d'une membrane à utiliser pour protéger la croissance contrôlée des tissus du ligament alvéolo-dentaire et/ou du cément, ledit film ou ladite membrane comprenant des éléments de renfort structuraux orientés polymères absorbables et/ou solubles, ladite orientation des éléments de renfort structuraux étant sélectionnée parmi une orientation biaxiale, une étoffe tissée, une étoffe tricotée et un non-tissé feutre.

A fibril (a bundle of microfibrils)

crystalline blocks

group of lamellae

Figure 1a

Transformation of lamellar structure to fibrillar structure

microfibrils

a crystalline block

an amorphous region

a = an intrafibrillar tie-molecule

b = an interfibrillar tie-molecule

Figure 1b
Microfibrils structure

f i b r i l s

Figure 1c

Figure 2

nonporous surface

porous core

nonporous surface

Figure 3.

the draw direction

the suture
loop

the hole

the film

Figure 4.